# FASCICULE DE BREVET EUROPEEN

(11) **EP 2 991 991 B1**
(45) Date de publication et mention de la délivrance du brevet: **26.09.2018**
(21) Numéro de dépôt: 14727606.7
(22) Date de dépôt: 30.04.2014
(51) Int. Cl.: C07D 493/04, C07B 63/04

(54) **PROCEDE DE STABILISATION D'UNE COMPOSITION CONTENANT AU MOINS UN PRODUIT DE DESHYDRATATION INTERNE D'UN SUCRE HYDROGENE, COMPOSITION OBTENUE ET SES UTILISATIONS**
VERFAHREN ZUR STABILISIERUNG EINER ZUSAMMENSETZUNG ENTHALTEND MINDENSTENS EIN DEHYDRATISIERUNGSPRODUKT EINES HYDROGENISIERTEN ZUCKERS, DADURCH HERGESTELLTE ZUSAMMENSETZUNG UND IHRE VERWENDUNG
METHOD FOR STABILIZING OF A COMPOSITION COMPRISING AT LEAST A DEHYDRATING PRODUCT OF A HYDROGENATED SUGAR, COMPOSITION PRODUCED ACCORDING TO THE METHOD, AND USE THEREOF

(30) Priorité: 02.05.2013 FR 1354034
(43) Date de publication de la demande: 09.03.2016
(73) Titulaire: Roquette Frères, 62136 Lestrem (FR)
(72) Inventeur: IBERT, Mathias, F-59930 La Chapelle D'armentieres (FR); WYART, Hervé, F-62149 Cuinchy (FR); FUERTES, Patrick, F-59160 Lomme (FR)
(74) Mandataire: Cabinet Plasseraud
(86) Numéro de dépôt international: PCT/FR2014/051043
(87) Numéro de publication internationale: WO 2014/177815

(56) Documents cités:
- WO-A1-03/043959
- WO-A1-2012/133850
- US-A1- 2003 032 832

## Description

La présente invention est relative à l'utilisation de monoéthanolamine, diéthanolamine, triéthanolamine ou un de leurs mélanges comme agent permettant d'améliorer la stabilité au stockage d'un isosorbide.

Valoriser nos ressources biologiques renouvelables est devenu un impératif écologique et économique majeur, face à l'épuisement et à la montée des prix des matières fossiles comme le pétrole. C'est dans ce contexte que s'inscrit le développement des 1,4:3,6-dianhydrohexitols.

Ces produits, également appelés isohexides, sont obtenus par déshydratation interne de sucres hydrogénés en C₆ (hexitols) tels que le sorbitol, le mannitol et l'iditol. Dans la présente demande, le terme « dianhydrohexitols » englobe l'isosorbide (1,4 - 3,6-dianhydro-sorbitol), l'isomannide (1,4 - 3,6 dianhydro-mannitol), l'isoidide (1,4 - 3,6-dianhydro-iditol) de formules suivantes, ainsi que les mélanges de ces produits :

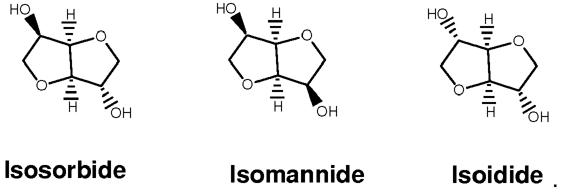

Il existe aujourd'hui un fort potentiel de développement industriel pour les isohexides, et notamment pour l'isosorbide dont on envisage l'utilisation pour la préparation :
- de 2-nitrate, 5-nitrate ou 2,5 dinitrate d'isosorbide, utiles dans le traitement thérapeutique de maladies, notamment cardiaques et/ou vasculaires ;
- de dérivés alcoylés, en particulier diméthylés, de l'isosorbide, utiles notamment comme solvants dans le cadre de la préparation de compositions pharmaceutiques ou cosmétologiques ;
- de dérivés d'isosorbide à destination de compositions détergentes pour carburants
- de dérivés alcoylés ou alcénylés utilisables comme plastifiants de polymères, d'adhésifs ou d'encres ;
- de biphosphites particuliers utilisables comme agents stabilisants de polymères ou lubrifiants ;
- d'articles à base d'alcool polyvinylique, de polyuréthannes, ou de polymères et copolymères tels que des polyesters et des polycarbonates ;
- de polycondensats biodégradables ;
- de laques aqueuses ou de compositions à action recouvrante et/ou colorante de surfaces.

Pour la majorité des applications précitées de l'isosorbide et autres produits de déshydratation interne de sucres hydrogénés, en particulier des autres isohexides, il est généralement requis d'appliquer un traitement de purification aux compositions résultant directement de l'étape de déshydratation proprement dite. Ceci, du fait notamment que tout sucre hydrogéné soumis à une telle étape (par exemple le sorbitol) est susceptible, lors de ladite étape, d'être transformé, outre en le produit de déshydratation recherché (par exemple l'isosorbide) en divers co-produits tels que :
- des isomères dudit produit recherché, par exemple des isomères de l'isosorbide tels que l'isomannide et l'isoidide,
- des produits moins déshydratés que le produit recherché ou que ses isomères, par exemple du sorbitan, du mannitan ou de l'iditan,
- des dérivés résultant de l'oxydation ou plus généralement de la dégradation des produits susmentionnés, ces dérivés pouvant inclure, par exemple lorsque le produit recherché est l'isosorbide, des co-produits de type déoxy monoanhydro hexitols, monoanhydro pentitols, monoanhydro tétritols, anhydro hexoses, hydroxyméthyl furfural, ou glycérine,
- des dérivés résultant de la polymérisation des produits susmentionnés, et/ou des espèces fortement colorées, de nature mal définie.

Il convient de rappeler que d'une manière générale, tout ou partie de ces différentes catégories de co-produits ou impuretés sont générées peu ou prou lors de l'étape de déshydratation proprement dite du sucre hydrogéné et ce, indépendamment des conditions et précautions mises en oeuvre en pratique lors de ladite étape, et par exemple indépendamment : de la nature et de la forme de présentation du catalyseur acide de déshydratation utilisé (acide minéral, acide organique, résine cationique, ...), ou de la quantité d'eau ou de solvant(s) organique(s) dans le milieu réactionnel initial, ou de la pureté de la composition de sucre hydrogéné, par exemple de sorbitol, utilisée comme matière première.

Aussi, il existe tout un pan de la littérature dédié à des technologies visant à obtenir des compositions issues de ladite étape de déshydratation, qui soient améliorées en terme de pureté et ce, en jouant sur les conditions réactionnelles lors de ladite étape, ou en appliquant un ou plusieurs traitement(s) de purification après ladite étape, ou encore en combinant ces différents moyens.

Dans la première catégorie, on peut citer le brevet CA 1 178 288 qui recommande (page 14, lignes 3-8) de conduire la réaction de déshydratation sous une atmosphère de gaz inerte pour éviter des réactions d'oxydation, notamment lorsque des températures et durées de réaction relativement importantes sont envisagées. Le brevet US 4 861 513 s'inscrit également dans cette lignée, en décrivant une réaction de déshydratation du sorbitol menée en présence simultanée de gaz inerte (azote) et d'un agent réducteur (hypophosphite de sodium) en vue de préparer des mélanges de polyols particuliers, lesquels présentent une faible teneur (10 à 26 % en poids sec) en dianhydrosorbitol.

Dans la deuxième catégorie, on peut se référer au brevet US 4 564 692 qui mentionne sans aucunement la détailler, la prépurification sur échangeurs d'ions et / ou charbon actif de compositions d'isosorbide ou d'isomannide puis, après concentration par évaporation et ensemencement de cristaux de l'isohexide recherché, la cristallisation dans l'eau de celui-ci. On peut également indiquer le brevet WO 01 / 94352 qui enseigne une purification par traitement avec un moyen d'échange ionique, puis un moyen de décoloration.

Enfin, dans la troisième catégorie, on peut mentionner le brevet GB 613 444 qui décrit l'obtention, par déshydratation en milieu eau / xylène, d'une composition d'isosorbide ensuite soumise à un traitement de distillation puis de recristallisation dans un mélange alcool / éther. Quant au brevet EP 0 323 994, il envisage la mise en oeuvre de catalyseurs de déshydratation particuliers (respectivement un halogénure d'hydrogène gazeux et du fluorure d'hydrogène liquide) associés avantageusement à des acides carboxyliques comme co-catalyseurs, puis la distillation des compositions brutes d'isosorbide ou d'isomannide ainsi obtenues.

En opposition avec cet état de la technique, la Demanderesse avait alors fait le double constat selon lequel le niveau de stabilité d'une composition d'isohexides n'était pas corrélé à son niveau de pureté, mais encore que la mise en oeuvre d'un agent comme l'azote gazeux ou le borohydrure de sodium telle que décrite dans l'art antérieur (au plus tard au moment de l'étape de distillation) ne permettait pas d'améliorer significativement cette stabilité.

Et c'est en poursuivant encore ses travaux que la Société Demanderesse avait alors trouvé de façon surprenante et inattendue, que seuls a) des agents stabilisants particuliers, en l'occurrence sous forme non gazeuse b) mis en oeuvre à un moment particulier du procédé de préparation, en l'occurrence postérieurement à l'étape proprement dite de distillation, permettaient de préparer des compositions d'isohexides dont le comportement au stockage, pour le moins à température ambiante ou modérée, était amélioré.

Cette invention à fait l'objet du brevet EP 1 446 373, protégeant un procédé de préparation d'une composition de produit de déshydratation interne d'un sucre hydrogéné, caractérisé par le fait qu'il comprend :
a) une étape de distillation d'un milieu contenant ledit produit de déshydratation interne en vue d'obtenir un distillat enrichi en ce produit,
b) éventuellement, au moins une étape subséquente de purification du distillat ainsi obtenu,
c) une étape subséquente de mise en présence du distillat obtenu lors de l'étape a), puis éventuellement soumis à l'étape b), avec un agent apte à améliorer la stabilité du produit de déshydratation interne majoritairement contenu dans le distillat, le dit agent n'étant pas sous forme gazeuse,
d) éventuellement, une étape subséquente de mise en forme de la composition résultante de produit de déshydratation interne d'un sucre hydrogéné.

Ce document relate un certain nombre de possibilités pour l'agent d'amélioration mis en oeuvre au cours de l'étape c), et qui peut être choisi parmi :
- les agents réducteurs, et notamment des composés à base de bore ou d'aluminium comme le borohydrure de sodium (NaBH4) ou l'aluminohydrure de lithium (LiAIH4) ;
- des composés à base de phosphore comme une phosphine ou un phosphite ;
- des agents anti-oxydants, et notamment des composés à base d'azote, en particulier des amines, aromatiques ou non, contenant ou non par ailleurs au moins une fonction alcool, comme l'hydroxylamine, la morpholine, et leurs dérivés ;
- des composés aromatiques, azotés ou non, contenant ou non au moins une fonction alcool, comme par exemple l'hydroquinone, le phénol, les tocophérols et leurs dérivés respectifs ;
- des composé anti-oxydant à base de phosphore ou de soufre tels que les phosphites, les phosphonites, les sulfites, les sels d'esters d'acide thiodipropionique et leurs mélanges ;
- des anti-acides ;
- des agents désactivateurs des métaux, comme des agents complexants ou chélatants des métaux d'origine naturelle ;
- des produits autorisés comme additifs alimentaires, en particulier ceux qualifiés de anti-oxygènes , correcteurs d'acidité ou séquestrants au sens de la réglementation européenne, comme l'acide ascorbique (vitamine C), l'acide érythorbique, l'acide lactique, l'acide citrique, l'acide gallique, les tocophérols, les dérivés (en particulier les sels) de l'ensemble de ces produits, le BHT, le butylhydroxyanisole (BHA) et les mélanges quelconques de ces produits.

La Société Demanderesse a alors démontré que par la mise en oeuvre d'un tel procédé, on aboutissait à des compositions particulièrement stables. Par composition stable au sens du document EP 1 446 373, on entend une composition qui, stockée dans une atmosphère non inerte pendant une durée d'au moins un mois et à une température de 40°C, présente à la fois une teneur en acide formique inférieure à 0,0005 % et une teneur globale en monoanhydrohexoses inférieure à 0,005 %, ces pourcentages étant exprimés en poids sec par rapport au poids sec de ladite composition.

Cette stabilité est, pour les compositions obtenues grâce au procédé précité, supérieure à 1 mois et peut atteindre au moins 2 mois, de préférence au moins 6 mois et plus préférentiellement encore au moins un an. A titre d'agent d'amélioration mis en oeuvre pendant l'étape c), le brevet EP 1 446 373 exemplifie le borohydrure de sodium, la morpholine, le BHT, la vitamine C, le borate de sodium, la soude, et le phosphate disodique, les meilleurs résultats (stabilité de 6 mois) étant obtenus pour le le borohydrure de sodium et la morpholine.

Or, travaillant à l'amélioration du procédé qu'elle avait développé, notamment en terme d'augmentation de la durée de stabilité d'une composition de produit de déshydratation interne d'un sucre hydrogéné, la Demanderesse est aujourd'hui parvenue à l'issue de nombreuses recherches à un tel résultat. Ce dernier est entre autre basé sur l'identification d'un paramètre ayant un rôle essentiel sur la stabilité précitée, mais aussi sur l'ajustement de ce paramètre.

En l'occurrence, la présente Demande illustre l'influence de la nature de l'agent d'amélioration introduit au niveau de l'étape c) du procédé selon le document EP 1 446 373, et que le choix particulier de la monoéthanolamine, de la diéthanolamine, de la triéthanolamine ou de leurs mélanges conduisait à des résultats particulièrement avantageux en terme d'allongement de la durée de stabilité des compositions envisagées. Ces résultats sont étayés par le suivi du pH au cours du temps de telles compositions, paramètre dont on sait qu'il est directement relié à la stabilité desdites compositions, comme indiqué dans le brevet précité. A ce titre, une dérive ou évolution notable du pH est significative d'une perte du degré de stabilité de telles compositions.

Aussi, un moyen pratique et fiable d'évaluer la stabilité de telles compositions est de suivre l'évolution de leur pH, après les avoir placées dans une enceinte thermostatée. Dans les exemples de la présente invention, c'est ainsi qu'a procédé la Demanderesse, fixant la température à 50°C de manière à être encore plus discriminante que dans le document EP 1 446 373. Elle a ainsi obtenu des pH particulièrement stables, en mettant en oeuvre la monoéthanolamine, la diéthanolamine et la triéthanolamine au niveau de l'étape c) du procédé selon la présente invention.

Par composition stable au sens de la présente Demande, on entend donc une composition dont le pH n'évolue pas de manière significative, à 50 °C, pendant une durée au moins égale à 1 mois, préférentiellement 3 mois, très préférentiellement 6 mois, de manière la plus préférée au moins égale à 1 an.

Aussi, l'objet de la présente invention consiste en l'utilisation de monoéthanolamine, diéthanolamine, triéthanolamine ou un de leurs mélanges comme agent permettant d'améliorer la stabilité au stockage d'un isosorbide, par la mise en présence de cet agent avec un distillat d'isosorbide obtenu par un procédé comprenant :
a) une étape de distillation d'un milieu contenant un isosorbide en vue d'obtenir un distillat enrichi en isosorbide,
b) éventuellement, au moins une étape subséquente de purification du distillat ainsi obtenu.

Eventuellement, on réalise une étape d) subséquente de mise en forme de la composition résultante de produit de déshydratation interne d'un sucre hydrogéné, i.e. d'isosorbide.

L'étape de mise en présence du distillat obtenu lors de l'étape a), puis éventuellement soumis à l'étape b), avec l'agent apte à améliorer la stabilité de l'isosorbide est nommée étape c).

De manière préférée, l'agent mis en oeuvre au niveau de l'étape c) est choisi parmi la diéthanolamine, la triéthanolamine et leurs mélanges.

L'agent mis en oeuvre au niveau de l'étape c) est utilisé à raison de 0,0001 à 2 %, préférentiellement de 0,001 à 2 %, plus préférentiellement de 0,002 à 1,5 %, ces pourcentages étant exprimés en poids sec d'agent d'amélioration par rapport au poids sec du produit de déshydratation interne de sucre hydrogéné alors majoritairement présent dans le distillat, i.e. l'isosorbide alors présent dans ce milieu.

Dans le cadre de l'invention, il convient de préciser que le milieu soumis à l'étape a) de distillation, peut être de nature très variée, y compris en termes de matière sèche, de température et / ou de pureté en le produit de déshydratation recherché. Il peut s'agir, selon une première variante, d'une composition d'isosorbide consistant en le milieu issu directement de la réaction de déshydratation proprement dite et présentant une pureté en produit recherché, i.e. isosorbide, de l'ordre de 50 à 80 %. Selon une autre possibilité, ladite composition peut, du fait notamment qu'elle résulte déjà d'une ou de plusieurs opérations antérieures de purification, notamment par distillation et / ou cristallisation, présenter une pureté en produit recherché, i.e. en isosorbide, supérieure à 80 %.

De manière avantageuse l'étape a) de distillation est suivie d'une étape b) de purification du distillat résultant.

Selon une première variante l'étape b) consiste en une étape de purification selon laquelle le distillat, généralement mis en solution, est traité avec au moins un moyen de purification choisi parmi les moyens de décoloration et les moyens d'échange ionique.

Par moyens de décoloration, on entend notamment le charbon actif sous forme granulaire ou pulvérulente et les résines d'adsorption. A titre d'exemple, on peut utiliser, seul ou en combinaison, du charbon actif granulaire comme le produit CECA DC 50, du charbon actif pulvérulent comme le produit NORIT SX+ et/ou une résine comme celles dénommées DUOLITE XAD 761, MACRONET MN-600 ou MACRONET MN-400.

Par moyens d'échange ionique, on entend notamment les résines anioniques, faibles ou fortes, et les résines cationiques, faibles ou fortes. A titre d'exemple, on peut utiliser, seule ou en combinaison, une résine anionique forte comme la résine AMBERLITE IRA 910 ou une résine cationique forte comme la résine PUROLITE C 150 S. Le moyen d'échange ionique peut avantageusement comprendre au moins une résine anionique et au moins une résine cationique. De préférence, ce moyen est composé d'un lit mixte de résines anionique(s) et cationique(s) ou d'une succession de résines cationique(s) puis anionique(s) ou d'une succession de résine (s) anionique (s) puis cationique (s).

De manière préférentielle, lors de l'étape b) du procédé conforme à l'invention, le distillat obtenu lors de l'étape a) est traité dans un ordre quelconque par au moins un charbon actif et par au moins une résine, ionique ou non ionique. De manière très avantageuse, ledit distillat est traité d'abord par un charbon actif puis par au moins une résine puis de nouveau par un charbon actif.

Selon une autre variante du procédé selon l'invention, la Société Demanderesse a trouvé qu'il était particulièrement avantageux que la composition soumise à l'étape b) de purification présente déjà certaines caractéristiques en termes de teneur maximale en impuretés particulières, par exemple en acide formique et en espèces de type monoanhydrohexoses. Elle a trouvé par ailleurs qu'une telle teneur pouvait notamment être garantie en soumettant directement le distillat obtenu lors de l'étape a) à ladite étape b) de purification.

Le procédé selon l'invention peut donc être caractérisé par le fait que le distillat soumis à ladite étape b) présente une teneur en acide formique inférieure à 0,002 % et une teneur en monoanhydrohexoses inférieure à 0,02 %, ces pourcentages étant exprimés en poids sec par rapport au poids sec du produit de déshydratation interne de sucre hydrogéné alors majoritairement présent dans ledit distillat, i.e. au poids sec d'isosorbide présent dans ledit distillat. Le distillat peut notamment présenter une teneur en acide formique inférieure à 0,0005 % et une teneur en monoanhydrohexoses inférieure à 0,005 %.

Selon une autre variante et de manière avantageuse, l'agent d'amélioration utilisé selon l'invention est mis en oeuvre directement après l'étape b) de purification et notamment, comme il sera exemplifié par ailleurs, introduit directement sur la solution aqueuse purifiée de produit de déshydratation interne issue de l'étape b), laquelle présente généralement une température au plus égale à 60 °C.

Quel que soit le mode de fonctionnement du procédé objet de l'invention, la Société Demanderesse a également trouvé qu'il pouvait être avantageux que tout ou partie de l'étape c), au cours de laquelle il y a présence de l'agent d'amélioration, se fasse au sein d'un milieu qui soit liquide et dont la température soit au moins égale à la température de ramollissement ou de fusion du produit de déshydratation interne recherché (i.e. l'isosorbide) mais inférieure à 140 °C environ. Cette température peut, en particulier dans le cas de l'isosorbide, se situer entre 60 et 135 °C.

Ces conditions améliorent la répartition homogène de l'agent d'amélioration au sein de la composition résultante, en particulier si celle-ci doit être refroidie puis mise en forme conformément à l'étape d) facultative.

Le mélange entre le distillat issu de l'étape a) avec l'agent stabilisant est extrêmement rapide car l'amine utilisée s'avère extrêmement soluble dans le distillat, de sorte que la durée de l'étape c) - pour peu qu'elle ne dure cependant pas moins d'une seconde environ - n'est pas critique pour le procédé en question.

Après l'étape c) et comme indiqué précédemment, la composition de produit de déshydratation interne d'un sucre hydrogéné, i.e. l'isosorbide, obtenue selon l'invention peut être mise en forme lors d'une étape d) ultérieure. Cette étape peut consister en une opération de pastillage ou d'écaillage de la masse cristallisée ou massé résultant du refroidissement, notamment par contact sur une surface froide, de la composition issue de l'étape c).

L'étape d) de mise en forme peut, si on le souhaite, être suivie d'une étape de broyage et/ou de tamisage et ce, avant toute étape de stockage et/ou d'ensachage de la composition ainsi obtenue.

Selon une autre variante, la composition de produit de déshydratation interne d'un sucre hydrogéné obtenue selon l'invention, i.e. d'isosorbide, peut, après l'étape c), être stockée en l'état, en particulier à l'état liquide ou pâteux, sans étape spécifique de mise en forme ultérieure.

La composition résultant du procédé selon l'invention peut par ailleurs avoir subi, à tout moment, une étape de concentration, en particulier une étape d'évaporation sous vide, ladite étape étant conduite dans les conditions les plus douces possible, notamment en termes de durée et de température. Cette étape consiste alors à concentrer ladite composition tout en la maintenant sous forme liquide, notamment à une teneur en matière sèche comprise entre 50 et 90 %, préférentiellement entre 75 et 88 %. Elle peut aussi consister à concentrer ladite composition de manière à obtenir un produit final sous forme sèche.

De manière préférée, il est le plus avantageux de mener ladite étape de concentration directement après l'étape c).

L'ensemble des étapes du procédé selon l'invention, obligatoires ou facultatives, qui viennent d'être décrites peuvent en outre, si on le souhaite, être menées sous atmosphère inerte, y compris l'étape c) caractéristique de la présente invention et / ou toute étape ultérieure, notamment de mise en forme, de stockage ou d'ensachage.
En suite de quoi, on dispose d'un nouveau moyen apte à fournir une composition d'un produit de déshydratation interne d'un sucre hydrogéné, i.e. d'isosorbide, dont la stabilité est améliorée, une telle stabilité étant requise quelles que soient les utilisations auxquelles on destine ladite composition et quelle que soit par ailleurs la pureté de ladite composition.

Aussi, la présente demande décrit également une composition contenant au moins un produit de déshydratation interne d'un sucre hydrogéné, caractérisée par le fait qu'elle contient, de 0,0001 à 2 %, préférentiellement de 0,001 à 2 %, plus préférentiellement de 0,002 à 1,5 %, d'un agent d'amélioration choisi parmi la monoéthanolamine, la diéthanolamine, la triéthanolamine et leurs mélanges, et qui est préférentiellement choisi parmi la diéthanolamine, la triéthanolamine et leurs mélanges, ces pourcentages étant exprimés en poids sec d'agent d'amélioration par rapport au poids sec du produit de déshydratation interne de sucre hydrogéné alors majoritairement présent dans ladite composition, par exemple de l'isosorbide alors présent dans ce milieu.

Cette composition dans une variante particulière est caractérisée en ce que le produit de déshydratation interne d'un sucre est l'isosorbide.

Dans une première variante où la composition est sous forme liquide, elle est caractérisée en ce qu'elle présente une matière sèche comprise entre 50 et 90 %, de préférence comprise entre 75 et 88 %.

Dans une seconde variante, ladite composition est sous forme solide (ladite composition présentant donc une matière sèche de 100 %).

De telles compositions peuvent notamment être utilisées pour la préparation de produits ou mélanges, polymériques ou non, biodégradables ou non, destinés aux industries chimiques, pharmaceutiques, cosmétologiques ou alimentaires.

La présente invention va être décrite de façon encore plus détaillée à l'aide des exemples qui suivent et qui ne sont aucunement limitatifs.

### EXEMPLES

### Exemple 1

Cet exemple porte sur la fabrication d'une composition d'isosorbide selon l'art antérieur (mise en oeuvre de phosphate disodique) et selon l'invention (utilisation de monoéthanolamine, diéthanolamine et triéthanolamine). Il illustre l'augmentation de la durée de stabilité desdites compositions dans le cadre de l'invention, à travers le suivi du pH de ces compositions au cours du temps.

Dans un réacteur agité double enveloppe, on introduit 1 kg d'une solution de sorbitol à 70 % de MS commercialisée par la Demanderesse sous l'appellation NEOSORB 70/02 et 7 g d'acide sulfurique concentré. Le mélange obtenu est chauffé sous vide (pression d'environ 100 mbars) pendant 5 heures de façon à éliminer l'eau contenue dans le milieu réactionnnel initial et celle provenant de la réaction de déshydratation du sorbitol.

Le brut réactionnel est ensuite refroidi vers 100 C puis neutralisé avec 11,4 g d'une solution de soude à 50 %. La composition d'isosorbide ainsi neutralisée est ensuite distillée sous vide (pression inférieure à 50 mbars).

Le distillat brut d'isosorbide, légèrement coloré (couleur jaune claire) est ensuite mis en solution dans le 2-propanol, à une température de 60 °C, de façon à obtenir une solution à 75 % de MS. Cette solution est ensuite refroidie lentement, en l'espace de 5 heures, jusqu' à une température de 10 °C une amorce d'isosorbide recristallisé est ajoutée à 40 °C.

Les cristaux sont ensuite essorés dans une essoreuse et lavés avec un peu de 2-propanol. Après séchage sous vide, les cristaux sont remis en solution dans l'eau de manière à obtenir une MS de 40 %.

Cette solution est ensuite percolée sur une colonne de charbon actif granulaire CPG 12-40 à une vitesse de 0,5 BV/h (Bed Volume ou Volume de lit /heure). La composition d'isosorbide décolorée ainsi obtenue est ensuite passée, à une vitesse de 2 BV/h successivement sur une colonne de résine cationique forte PUROLITE C 150 S puis une colonne de résine anionique forte AMBERLITE IRA 910. Cette solution est ensuite traitée par du charbon actif en poudre de type NORIT SX+ à 20°C pendant 1 heure. Le charbon actif est mis en oeuvre à raison de 0,5 % exprimé en poids sec / poids sec de solution.

On introduit alors dans ladite composition l'agent à tester, soit :
- 0,005 % de phosphate disodique (poids sec / poids sec d'isosorbide contenu dans la composition) pour l'essai n°1 ;
- 0,0025 % de monoéthanolamine (poids sec / poids sec d'isosorbide contenu dans la composition), pour l'essai n°2 ;
- 0,0025 % de diéthanolamine (poids sec / poids sec d'isosorbide contenu dans la composition), pour l'essai n°3 ;
- 0,0025 % de triéthanolamine (poids sec / poids sec d'isosorbide contenu dans la composition), pour l'essai n°4.

Après filtration, la solution d'isosorbide est concentrée sous vide. La masse fondue obtenue cristallise au refroidissement sous forme d'un massé de gros cristaux que l'on broye ensuite pour obtenir une poudre de couleur blanche présentant une humidité de 0,2 %.

200 g de ce massé d'isosorbide sont directement introduits dans un récipient en verre d'une contenance de 500 ml qui, après avoir été fermé hermétiquement, est placé dans une étuve maintenue à 50 °C.

Pour chacun des flacons correspondant aux essais n°1, 2, 3 et 4, on suit l'évolution du pH au cours du temps. Les résultats apparaissent dans le tableau 1. La mesure de pH est effectuée sur un pHmètre de marque RADIOMETER ANALYTICAL PHM 220 équipé d'une électrode combinée à fil Ag / AgCl de marque METTLER TOLEDO, préalablement étalonnée à l'aide de solutions tampon pH 7 et 4. Le pH est mesuré sur un échantillon de produit dissous à 40% en poids de matière sèche dans de l'eau osmosée.

On vérifie bien que, seuls les essais n°2, 3 et 4 présentent un pH avantageusement stable dans le temps pendant au moins 3 mois, cette période pouvant atteindre 6 mois dans le cas des essais n°3 et 4 correspondant à la diéthanolamine et à la triéthanolamine.

**Tableau 1**

| Essai n° | Agent selon l'étape c) | | pH | | | | | | | | |
|---|---|---|---|---|---|---|---|---|---|---|---|
| | Nature | Quantité (% sec / sec) | t=0 | t=1 mois | t=1,5 mois | t=2 mois | t=2,5 mois | t=3 mois | t=4 mois | t=5 mois | t=6 mois |
| 1 | phosphate disodique | 0,005 | 7,9 | 7,8 | 7,9 | 7,7 | 3,3 | | | | |
| 2 | monoéthanol amine | 0,0025 | 7,4 | 7,3 | | 6,8 | | 6,9 | 3,2 | | |
| 3 | diéthanol amine | 0,0025 | 7,8 | 7,6 | | 7,5 | | 7,5 | 7,5 | 7,5 | 7,5 |
| 4 | triéthanol amine | 0,0025 | 7,9 | 7,7 | | 8 | | 7,9 | 7,7 | 7,7 | 7,8 |

### Exemple 2

Cet exemple porte sur la fabrication d'une composition d'isosorbide selon l'art antérieur (mise en oeuvre de soude, d'acide ascorbique, de tocophérol, de metaborate de sodium, de morpholine, de butyl hydroxyl tolune) et selon l'invention (utilisation de diéthanolamine et triéthanolamine). Il illustre l'augmentation de la durée de stabilité desdites compositions dans le cadre de l'invention, à travers le suivi du pH selon un protocole identique à celui décrit dans l'exemple précédent.

La seule différence réside en ce que l'étuve est maintenue à 60 °C, ce qui rend les tests largement plus discriminants, des différences pouvant être observées sur une période de quelques jours.

Les résultats figurent dans le tableau 2.

On montre bien ainsi que les meilleures stabilités sont obtenues pour la diéthanolamine et la triéthanolamine.

**Tableau 2**

| Stabilisant | DEA | TEA | NaOH | Acide Ascorbique | Tocophérol | Metaborate de Na | Morpholine | BHT |
|---|---|---|---|---|---|---|---|---|
| T=0 | 8,3 | 7,7 | 9,1 | 4,9 | 5,3 | 8,5 | 7,9 | 6,9 |
| T=2 jours | 8,6 | 8,0 | 8,0 | 4,3 | 3,5 | 8,6 | 7,8 | 3,9 |
| T=3 jours | 8,5 | 8,0 | 8,0 | 3,9 | 3,3 | 7,9 | 7,2 | 3,5 |
| T=5 jours | 8,0 | 8,0 | 7,4 | 3,5 | 3,2 | 7,7 | 7,1 | 3,4 |

## Revendications

1. Utilisation de monoéthanolamine, diéthanolamine, triéthanolamine ou un de leurs mélanges comme agent permettant d'améliorer la stabilité au stockage d'un isosorbide, par la mise en présence de cet agent avec un distillat d'isosorbide obtenu par un procédé comprenant les étapes suivantes :
a) une étape de distillation d'un milieu contenant un isosorbide en vue d'obtenir un distillat enrichi en isosorbide,
b) éventuellement, au moins une étape subséquente de purification du distillat ainsi obtenu.

2. Utilisation selon la revendication 1, **caractérisée en ce que** l'agent permettant d'améliorer la stabilité au stockage d'un isosorbide est choisi parmi la diéthanolamine, la triéthanolamine et leurs mélanges.

3. Utilisation selon l'une quelconque des revendications 1 ou 2, **caractérisée en ce que** l'agent permettant d'améliorer la stabilité au stockage d'un isosorbide et mis en présence avec un distillat d'isosorbide est utilisé à raison de 0,0001 à 2 %, préférentiellement de 0,001 à 2 %, plus préférentiellement de 0,002 à 1,5 %, ces pourcentages étant exprimés en poids sec d'agent par rapport au poids sec du produit d'isosorbide alors majoritairement présent dans le distillat.

4. Utilisation selon l'une quelconque des revendications 1 à 3, **caractérisée par le fait que** l'étape a) de distillation est suivie d'une étape b) de purification, de préférence une étape b) de purification au cours de laquelle le distillat est traité, dans un ordre quelconque, par au moins un charbon actif et par au moins une résine, ionique ou non ionique.

5. Utilisation selon l'une quelconque des revendications 1 à 4, **caractérisée par le fait que** le procédé comprend au moins une étape de concentration.

## Patentansprüche

1. Verwendung von Monoethanolamin, Diethanolamin, Triethanolamin oder deren Mischung als Mittel, welches es ermöglicht, die Lagerungsstabilität eines Isosorbids durch das Zusammenbringen dieses Mittels mit einem Isosorbiddestillat zu verbessern, wobei das Isosorbiddestillat durch ein Verfahren erhalten wird, welches die folgenden Schritte umfasst:
a) einen Schritt der Destillation eines ein Isosorbid enthaltenden Mediums mit dem Ziel, ein mit Isosorbid angereichertes Destillat zu erhalten,
b) gegebenenfalls wenigstens einen nachfolgenden Schritt der Reinigung des so erhaltenen Destillats.

2. Verwendung nach Anspruch 1, **dadurch gekennzeichnet, dass** das Mittel, welches es ermöglicht, die Lagerungsstabilität eines Isosorbids zu verbessern, aus Diethanolamin, Triethanolamin oder deren Mischungen gewählt ist.

3. Verwendung nach einem der Ansprüche 1 oder 2, **dadurch gekennzeichnet, dass** das Mittel, welches es ermöglicht, die Lagerungsstabilität eines Isosorbids zu verbessern, und welches mit einem Isosorbiddestillat zusammengebracht wird, in einer Menge von 0,0001 bis 2 %, bevorzugt von 0,001 bis 2 %, stärker bevorzugt von 0,002 bis 1,5 % verwendet wird, wobei diese Prozentsätze als Trockengewicht des Mittels in Bezug auf das Trockengewicht des mehrheitlich in dem Destillat vorhandenen Isosorbidproduktes ausgedrückt sind.

4. Verwendung nach einem der Ansprüche 1 bis 3, **dadurch gekennzeichnet, dass** auf den Schritt a) der Destillation ein Schritt b) der Reinigung folgt, bevorzugt ein Schritt b) der Reinigung, in dessen Verlauf das Destillat in einer beliebigen Reihenfolge mit wenigstens einer Aktivkohle und wenigstens einem ionischen oder nicht ionischen Harz behandelt wird.

5. Verwendung nach einem der Ansprüche 1 bis 4, **dadurch gekennzeichnet, dass** das Verfahren wenigstens einen Konzentrationsschritt umfasst.

## Claims

1. Use of monoethanolamine, diethanolamine, triethanolamine, or a mixture thereof, as an agent capable of improving the stability of an isosorbide by bringing this agent into contact with an isosorbide distillate obtained by a method comprising:
a) a step of distilling a medium containing said isosorbide in order to obtain a distillate enriched in isosorbide,
b) optionally, at least one subsequent step of purifying the distillate thus obtained.

2. The use as claimed in claim 1, **characterized in that** the agent capable of improving the stability of an isosorbide is chosen from diethanolamine and triethanolamine, and mixtures thereof.

3. The use as claimed in either one of claims 1 and 2, **characterized in that** the agent capable of improving the stability of an isosorbide and brought into contact with an isosorbide distillate is used in a proportion of from 0.0001% to 2%, preferentially from 0.001% to 2%, more preferentially from 0.002% to 1.5%, these percentages being expressed by dry weight of improving agent relative to the dry weight of the internal dehydration product of hydrogenated sugar then predominantly present in the distillate.

4. The use as claimed in any one of claims 1 to 3, **characterized in that** the distilling step a) is followed by a purifying step b), preferably a purifying step b) during which the distillate is treated, in any order, with at least one active carbon and with at least one ionic or nonionic resin.

5. The use as claimed in any one of claims 1 to 4, **characterized in that** the method comprises at least one concentrating step.
